Europäisches Patentamt

European Patent Office

Office européen des brevets

(19)

(11) Numéro de publication : **0 070 437 B1**

## FASCICULE DE BREVET EUROPÉEN

(12)

(45) Date de publication du fascicule du brevet :
20.02.85

(51) Int. Cl.⁴ : **A 23 G  1/02, C 07 D473/12**

(21) Numéro de dépôt : **82105906.0**

(22) Date de dépôt : **02.07.82**

(54) **Procédé d'élimination des composés stimulants du cacao.**

(30) Priorité : **22.07.81 CH 4778/81**

(43) Date de publication de la demande :
**26.01.83 Bulletin 83/04**

(45) Mention de la délivrance du brevet :
**20.02.85 Bulletin 85/08**

(84) Etats contractants désignés :
**DE FR GB**

(56) Documents cités :
**EP-A- 0 014 362
DE-A- 2 342 177
FR-A- 2 398 463
GB-A-   577 396**

(73) Titulaire : **SOCIETE DES PRODUITS NESTLE S.A.
Case postale 353
CH-1800 Vevey (CH)**

(72) Inventeur : **Magnolato, Danièle
Ch. du Jordil 2
CH-1807 Blonay (CH)**
Inventeur : **Isely, Alain
CH-1171 Montherod (CH)**

## Description

L'invention concerne un procédé de traitement du cacao pour en éliminer les composés stimulants ou purines comme la théobromine et la caféine.

On sait que les fèves de cacao contiennent de 1 à 2 % en poids de théobromine et environ dix fois moins de caféine. On a désiré depuis longtemps préparer du cacao dépurinisé qu'il soit destiné à entrer dans la fabrication de boissons ou de chocolat. Ainsi les brevets des Etats-Unis nos 1 855 026, 1 073 441 et 1 925 326 proposent l'extraction des purines par des solvants chlorés cependant que le brevet des Etats-Unis n° 2 118 129 décrit une méthode de récupération de la théobromine par mise en solution aqueuse et séparation des solides du cacao par formation d'un sel obtenu en ajoutant un oxyde alcalin. Il s'agit dans les deux cas de traitements par voie chimique qui sont jugés indésirables.

Enfin, la demande de brevet allemand n° 2 342 177 décrit l'extraction de la théobromine par l'eau chaude, mais, d'une part, la théobromine extraite n'est pas récupérée et, d'autre part, la masse de cacao obtenue après séparation de la solution aqueuse est impropre à la fabrication de chocolat ou de boisson cacaotée de qualité.

Nous avons trouvé un procédé permettant la dépurinisation complète du cacao par voie purement physique, la récupération quantitative de la théobromine et de la caféine et l'utilisation de cacao dépurinisé dans la fabrication de boissons cacaotées et de chocolat de qualité organoleptique satisfaisante. Lee procédé selon l'invention est caractérisé par les étapes suivantes :

a) on extrait les fèves de cacao vertes ou torréfiées à l'eau à une température de 40 à 60 °C,

b) on traite l'extrait aqueux par un adsorbant sous forme divisée à base de particules de caroube désucrées et activées de manière à adsorber les purines,

c) on sèche les fèves, on les torréfie et

d) on récupère les purines et on régénère l'adsorbant par lavage de celui-ci à une température de 80 à 100 °C.

Dans une variante préférée, on réhydrate les fèves séchées dans l'étape c) avec l'extrait aqueux dépurinisé, de préférence après concentration de celui-ci, de manière à réincorporer les solides extraits autres que la théobromine et la caféine. Cette réincorporation des solides extraits permet d'obtenir un cacao plus équilibré du point de vue organoleptique convenant particulièrement à la fabrication de chocolat.

Les fèves de cacao mises en œuvre peuvent être vertes ou torréfiées, de préférence sous forme concassées et dépelliculées. L'utilisation de fèves entières est également possible mais elle nécessite de plus longues durées d'extraction. On préfère utiliser des fèves vertes car cela permet d'éviter une opération de torréfaction.

L'adsorbant est constitué de particules de caroube désucrées et activées. Il est constitué par les résidus fibreux des gousses de caroube dont on a extrait les sucres à l'eau chaude. Le caroubier ou locuste, Ceratonia siliqua (famille des légumineuses) est un arbre pouvant aller jusqu'à 20 m de haut, originaire de Syrie et actuellement cultivé dans les pays méditerranéens. Son fruit contient des graines dont l'endosperme constitue la source de la gomme de caroube. Le péricarpe ou gousse est soit jeté, soit utilisé pour produire un sirop de basse qualité (sirop de caroube), ou encore dans l'alimentation animale. Les résidus sont habituellement jetés.

Ainsi la matière première utilisée pour préparer l'adsorbant est constituée soit des gousses, soit des résidus provenant de l'extraction des sucres. Il est donc avantageux de traiter ce matériau pour le débarrasser des impuretés, des sucres et des arômes de caroube par toute méthode convenable. On procède habituellement à une mouture grossière du matériau. Dans le cas où on traite les gousses non désucrées, une méthode commode consiste à les refroidir par exemple à − 40 °C et à les moudre. Les particules de gousses de caroube ou de résidus partiellement désucrés subissent un traitement à l'eau chaude pour éliminer les sucres. On peut utiliser, par exemple une batterie de cellules. d'extraction en ligne et extraire les sucres à l'eau chaude, par exemple à 95 °C. Les particules de caroube sont ensuite avantageusement séchées par exemple sous un léger vide, ce qui permet leur désodorisation.

Ces particules sont ensuite traitées par un acide, puis éventuellement désodorisées, par exemple par entraînement à la vapeur. On peut employer tout acide convenable, comme par exemple l'acide chlorhydrique, sulfurique ou phosphorique à l'état dilué ou concentré. Un traitement à l'acide chlorhydrique dilué pendant 1 à 3 heures à température ambiante convient.

Pour des raisons pratiques, on préfère utiliser un matériau de granulométrie relativement régulière. Le matériau subit donc un tamisage, les particules retenues étant avantageusement de dimension 0,3 à 5 mm et de préférence de 0,5 à 4 mm.

Suivant d'autres caractéristiques, les différentes étapes du procédé selon l'invention seront présentées ci-après sous forme de chapitre repéré par un titre :

(A) Extraction : Les fèves vertes ou torréfiées sous forme concassées et dépelliculées sont extraites à l'eau chaude (T : 40-60 °C) pendant 3-6 h, dans un système slurry, avec agitation. Le rapport pondéral fèves/eau se situe entre 1/30 et 1/50.

La perte en matières sèches au cours de l'extraction est de 15-18 %. Outre la théobromine et la caféine qui représentent respectivement 1,2-1,5 % et 0,2-0,5 % du poids sec des fèves, l'extrait aqueux contient principalement des hydrates de carbone, des sels minéraux, des acides organiques et des polyphénols.

La durée de l'extraction varie inversément à la température. On a cependant constaté que les caractéristiques organoleptiques d'un chocolat obtenu à partir de fèves traitées à 90 °C n'étaient pas satisfaisantes.

(B) Adsorption : La théobromine et la caféine peuvent être adsorbées quantitativement et sélectivement sur des particules de caroubes désucrées et activées comme indiqué ci-dessus. Pour ce faire, l'extrait aqueux des fèves contenant 15-18 % des matières sèches du cacao mis en œuvre est mis au contact de l'adsorbant, soit selon un procédé en colonne, soit en slurry avec agitation. Le rapport pondéral adsorbant/extrait se situe entre 1/30 et 1/50. La durée du traitement est de 2-4 h à température ambiante. L'absence de théobromine et de caféine dans l'éluat est vérifiée par chromatographie liquide à haute performance (HPLC) ; la sélectivité de l'adsorption est vérifiée par détermination de la matière sèche de l'éluat. La variation du taux de matière sèche de l'extrait correspond à la somme de la théobromine et de la caféine contenues dans les fèves (en général 1,5-2,0 % selon le type de fèves considéré).

(C) Séchage et torréfaction : Les fèves extraites à l'eau chaude sont séchées dans une étuve à circulation d'air, à une température de 60-80 °C, pendant 2-4 h. La torréfaction des fèves se fait selon un procédé classiquement employé pendant 10-20 min. à une température de 115-125 °C.

(D) Régénération : La caroube servant d'adsorbant à la caféine et à la théobromine peut être régénérée, après que la caféine et la théobromine aient été désorbées quantitativement par lavage à l'eau chaude à une température de 80-100 °C, pendant 1 h en slurry, le rapport pondéral adsorbant/eau étant de 1/30 à 1/100, effectué en 2 ou 3 traitements successifs. La caroube ainsi régénérée peut être réutilisée. On constate toutefois une perte d'environ 5 % des capacités d'adsorption après chaque cycle. Cette perte peut être compensée par l'ajout d'une faible quantité de caroube fraîche.

La variante décrite ci-dessus ne comprend pas la réincorporation des solides non puriniques extraits à l'étape (A). Les fèves obtenues, ne contenant ni théobromine ni caféine, conviennent à la fabrication de poudre de cacao entrant dans la confection de boissons cacaotées avec ou sans céréales maltées. Cette poudre peut également être solubilisée par une alcalinisation de manière classique pour l'obtention de poudre instantanément soluble. Ce cacao ne convient pas à la fabrication d'un chocolat de qualité satisfaisante à cause de son caractère doux et de la diminution de son caractère cacaoté.

Selon une variante préférentielle, l'extrait aqueux dépurinisé dans l'étape (B) est concentré puis sert à réhydrater les fèves séchées par l'étape (C) avant leur torréfaction selon les caractéristiques décrites ci-après pour les étapes (E), (F) et (G) :

(E) Concentration : L'extrait aqueux débarrassé de la théobromine et de la caféine qu'il contenait est concentré sous un vide de 700-740 mm Hg et une température de 40-60 °C. La concentration se poursuit jusqu'à obtention d'un volume valant du 1/40e au 1/20e du volume de départ.

(F) Réhydratation (ou réincorporation des solides autres que la théobromine et la caféine extraits en (B)). Les fèves séchées en (C) sont placées au contact du concentrat obtenu en (E) pendant 8-12 h, à une température se situant entre 20 et 30 °C. La réhydratation permet la réincorporation de 13-16 % des solides.

(G) Séchage (partiel) : Les fèves réhydratées et ayant récupéré les solides extraits sans théobromine ni caféine sont séchées pendant 1-2 h, à une température de 70-80 °C dans une étuve à circulation d'air. Un séchage complet n'est pas souhaitable ; il peut provoquer le développement d'un goût « brûlé » au cours de la torréfaction.

L'expérience a montré lors de dégustations que la réincorporation des solides extraits (étape (F)) était nécessaire à la conservation des caractéristiques organoleptiques du cacao, en particulier pour les notes « cacaoté » et « arôme ».

Le chocolat préparé à partir des fèves de cacao traitées selon la variante préférentielle du procédé décrite ci-dessus a été soumis à l'appréciation d'un jury de dégustateurs.

Malgré une légère diminution du caractère cacaoté, une diminution très faible des caractères acide et amer, une constance des caractères doux et astringent et un très léger goût étranger, le chocolat a été jugé tout à fait satisfaisant.

Les exemples suivants illustrent la mise en œuvre du procédé selon l'invention. Dans ceux-ci, les pourcentages sont exprimés en valeur pondérale.

Exemple 1

100 g de fèves de type BAHIA, torréfiées, concassées et dépelliculées, contenant 1,3 % de théobromine et 0,2 % de caféine sont extraites par 4 000 ml d'eau à 50 °C pendant 4 h en slurry. La perte en matière sèche au cours de l'extraction est de 16 %. Les fèves sont récupérées par tamisage et séchées pendant 4 h à 70 °C. L'extrait contenant la théobromine, la caféine et les autres solides extraits est passé à travers une colonne contenant 100 g de particules de caroubes désucrées et activées, avec un débit de 15 ml/mn. La théobromine et la caféine sont adsorbées quantitativement sur la caroube. L'éluat contenant 14,5 g de matières sèches est constitué principalement d'hydrates de carbone, sels minéraux, acides organiques et polyphénols. Il est concentré sous un vide de 720 mm Hg et une température de 50 °C jusqu'à obtention d'un volume de 100 ml. Ce concentrat est placé au contact des fèves pendant 12 h à 25 °C de manière à réintroduire les solides extraits. Les fèves ne contenant ni théobromine ni caféine sont ensuite séchées pendant 2 h à 70 °C dans une étuve à circulation d'air et torréfiées à 120 °C pendant 15 mm.

Exemple 2

250 g de fèves vertes du type GHANA concassées et dépelliculées contenant 1,5 % de théobromine et 0,15 % de caféine sont extraites par 5 000 ml d'eau à 100 °C pendant 2h, en slurry. Les fèves traitées sont séparées par tamisage et lavées à l'eau froide pour éliminer les traces de théobromine et de caféine susceptibles de se trouver en surface. Elles sont ensuite séchées pendant 2 h à 70 °C dans une étuve sans circulation d'air et torréfiées 15 mn à 120 °C. Les fèves ainsi traitées, ne contenant ni théobromine ni caféine et ayant perdu 15 % de leur matière sèche sont dégustées sous la forme de ganaches (mélange de cacao, de sucre glace et d'eau, sorte de chocolat grossier obtenu avant conchage). Le produit obtenu est fade et a perdu une grande partie de sa note cacaotée. Cet exemple indique d'une part qu'une extraction à 100 °C est préjudiciable au goût du produit final et d'autre part que les solides extraits jouent un rôle non négligeable sur la qualité organoleptique.

Exemple 3

250 g de fèves vertes du type GHANA identiques à celles décrites ci-dessus sont extraites dans les mêmes conditions que dans l'exemple 2.

Les fèves extraites sont lavées à l'eau froide avant d'être séchées pendant 4 h à 70 °C dans une étuve avec circulation d'air. L'extrait aqueux des fèves est mis au contact de caroube pendant 1 h à température ambiante en slurry, pour un rapport adsorbant/extrait de 1/6.

Dans ces conditions, les 3,75 g de théobromine et les 0,38 g de caféine extraits des fèves sont adsorbés quantitativement et sélectivement sur les 850 g d'adsorbant.

L'extrait aqueux est séparé de la caroube par filtration ; il est ensuite concentré par évaporation sous vide de manière à ramener le volume de l'extrait à 250 ml. Ce concentrat est réincorporé aux fèves traitées et séchées pendant 10 h à température ambiante. Comme dans l'exemple précédent, les fèves sont ensuite séchées 2 h à 70 °C avant d'être torréfiées 15 mn à 120 °C. Le cacao ainsi traité ne contenant ni théobromine ni caféine mais dans lequel on a réincorporé les solides extraits est dégusté sous forme de ganache. Le résultat du test organoleptique est sensiblement identique à celui de l'exemple précédent. Ceci montre que même en réincorporant les solides extraits, le goût du produit traité n'est pas satisfaisant. Ceci montre l'importance de la température d'extraction sur le goût du produit traité. On ne peut espérer obtenir un cacao exempt de théobromine et de caféine acceptable lorsqu'il a été extrait à 100 °C. Comme le montrait l'exemple 1, une extraction plus longue mais à une température plus basse (proche de 50 °C) est indispensable pour l'obtention d'un produit acceptable du point de vue organoleptique et pouvant servir de base à la fabrication de chocolat.

Exemple 4

10 g de fèves de cacao vertes du type GHANA contenant 1,33 % de théobromine sont extraites par 200 ml d'eau à 60 °C pendant 4 h. L'extrait aqueux contient 133 mg de théobromine, soit la totalité de la théobromine contenue initialement dans les fèves. Cet extrait aqueux est placé au contact de 30 g d'adsorbant. Après 1 h de réaction en slurry, avec agitation, à température ambiante, 131 mg soit 99 % de la théobromine sont adsorbés.

L'extrait aqueux (200 ml) est séparé de la caroube par filtration ; il est ensuite concentré par évaporation sous vide de manière à ramener le volume à 20 ml. Les fèves sont séchées 3 h à 70 °C avec circulation d'air ; elles sont ensuite mises au contact de l'extrait pendant 12 h à température ambiante. Le cacao ainsi traité ne contenant ni théobromine ni caféine mais dans lequel on a réincorporé les solides extraits est ensuite partiellement séché pendant 2 h à 70 °C avant d'être torréfié 20 mn à 120 °C. Le cacao ainsi traité est dégusté sous forme de ganaches. Le résultat du test organoleptique montre que le produit est tout à fait acceptable bien que légèrement inférieur à celui obtenu à partir de fèves torréfiées du type BAHIA (ex. 1) (moins cacaoté).

Les 30 g d'adsorbant contenant 131 mg de théobromine sont mis au contact de 1 500 ml d'eau à 90 °C : après 1 h sous agitation, 77 mg soit 59 % de la théobromine sont désorbés. La caroube est séparée de l'extrait par filtration et replacée au contact d'une nouvelle charge de 1 500 ml d'eau à 90 °C. La théobromine désorbée après 1 h sous agitation est de 51 mg soit 39 %. Il reste alors 3 mg de théobromine adsorbés sur la caroube, soit 2 % de la théobromine de départ qui peuvent être désorbés par lavage avec 1 500 ml d'eau à température ambiante.

L'adsorbant ainsi régénéré est placé au contact de 200 ml d'une nouvelle solution d'extrait de fèves contenant 133 mg de théobromine. Après 1 h de réaction dans les mêmes conditions, 111 mg de théobromine sont adsorbés ce qui correspond à une perte de capacité de fixation de 15 %. Au cours du 3e cycle, la caroube fixe 105 mg de théobromine ce qui correspond à une perte de capacité de 5 %. Cette perte de 5 % se stabilise au cours des cycles suivants. Pour retrouver la capacité d'adsorption initiale il convient d'ajouter 15 puis 5 % du poids de caroube initial sous forme de caroube fraîche.

**Revendications**

1. Procédé de traitement du cacao pour en éliminer les composés stimulants ou purines caractérisé par les étapes suivantes :

a) on extrait les fèves de cacao vertes ou torréfiées à l'eau à une température de 40 à 60 °C,

b) on traite l'extrait aqueux par un adsorbant sous forme divisée à base de particules de caroube désucrées et activées de manière à adsorber les purines,

c) on sèche les fèves, on les torréfie et

d) on récupère les purines et on régénère l'adsorbant par lavage de celui-ci à une température de 80 à 100 °C.

2. Procédé selon la revendication 1, caractérisé par le fait qu'entre le séchage et la torréfaction de l'étape c), on concentre l'extrait aqueux dépurinisé et on réhydrate les fèves avec l'extrait concentré de manière à réincorporer les solides extraits autres que la théobromine et la caféine.

3. Procédé selon la revendication 1 ou 2, caractérisé par le fait que dans l'étape b), on traite l'extrait aqueux contenant 15 à 18 % en poids des matières sèches du caco mis en œuvre par mise au contact de l'adsorbant en colonne ou en slurry à température ambiante pendant 2 à 4 heures dans un rapport pondéral adsorbant/extrait de 1/30 à 1/50.

4. Procédé selon la revendication 2, caractérisé par le fait qu'on concentre l'extrait aqueux dépurinisé sous un vide de 700 à 740 mm de mercure et à une température de 40 à 60 °C jusqu'à obtenir un volume représentant du 1/40e au 1/20e du volume de départ.

5. Procédé selon la revendication 4, caractérisé par le fait qu'on réhydrate les fèves séchées dans l'étape c) par mise en présence de l'extrait dépurinisé concentré pendant 8 à 12 h à une température de 20 à 30 °C.

6. Procédé selon la revendication 5, caractérisé en ce qu'on sèche partiellement les fèves réhydratées pendant 1 à 2 h à une température de 70 à 80 °C.

7. Utilisation du produit obtenu par la mise en œuvre du procédé selon l'une des revendications 1 à 6 pour fabriquer du chocolat.

## Claims

1. A process for treating cocoa to remove theobromine and caffeine therefrom, characterised by the following stages :

a) green or roasted cocoa beans are extracted with water at a temperature of from 40 to 60 °C,

b) the aqueous extract is treated with an adsorbent, in subdivided form, based on desugared and activated carob particles in order to adsorb theobromine and caffeine,

c) the beans are dried and roasted and

d) theobromine and caffeine are recovered and the adsorbent is regenerated by washing at a temperature of from 80 to 100 °C.

2. A process according to claim 1, characterised in that between the drying and roasting of stage c), the depurinised aqueous extract is concentrated and the beans are rehydrated with the concentrated extract in order to reincorporate the extracted solids, other than theobromine and caffeine.

3. A process according to claim 1 or 2, characterised in that in stage b), the aqueous extract containing from 15 to 18 % by weight of dry matter of the cocoa used is contacted with the adsorbent in a column or in the form of a slurry at ambient temperature over a period of 2 to 4 hours in a weight ratio of adsorbent to extract of from 1 : 30 to 1 : 50.

4. A process according to claim 2, characterised in that the depurinised aqueous extract is concentrated under a vacuum of from 700 to 740 mm of mercury and at a temperature of from 40 to 60 °C to obtain a volume representing from 1/40 to 1/20 of the starting volume.

5. A process according to claim 4, characterised in that the beans which are dried in stage c) are rehydrated by contact with the concentrated and depurinised extract over a period of 8 to 12 hours at a temperature of from 20 to 30 °C.

6. A process according to claim 5, characterised in that the rehydrated beans are partly dried over a period of from 1 to 2 hours at a temperature of from 70 to 80 °C.

7. Use of the product obtained by carrying out the process according to anyone of claims 1 to 6 for manufacturing chocolate.

## Ansprüche

1. Verfahren zur Behandlung von Kakao zur Entfernung von stimulierenden Verbindungen oder Purinen, gekennzeichnet durch die folgenden Schritte :

a) man extrahiert grüne oder geröstete Kakaobohnen mit Wasser einer Temperatur von 40 bis 60 °C,

b) man behandelt den wäßrigen Extrakt mit einem Adsorbens in zerteilter Form auf der Basis von Johannisbrotteilchen, die entzuckert und so aktiviert sind, daß sie die Purine adsorbieren,

c) man trocknet die Bohnen, man röstet sie und

d) man gewinnt die Purine und man regeneriert das Adsorbens durch deren Auswaschen bei einer Temperatur von 80 bis 100 °C.

2. Verfahren nach Anspruch 1, dadurch gekennzeichnet, daß man zwischen dem Trocknen und dem Rösten in Stufe c) den von Purinen befreiten wäßrigen Extrakt konzentriert und die Bohnen mit dem konzentrierten Extrakt so rehydratisiert, daß die extrahierten Feststoffe mit Ausnahme von Theobromin und Koffein wieder eingeführt werden.

3. Verfahren nach Anspruch 1 oder 2, dadurch gekennzeichnet, daß man in Stufe b) einen wäßrigen Extrakt, der 15 bis 18 Gew.-% Trockenfeststoffe des eingesetzten Kakaos enthält, so behandelt, daß man ihn mit dem Adsorbens in einer Säule oder als Aufschlämmung bei Umgebungstemperatur für einen Zeitraum vom 2 bis 4 Std. bei einem Gewichtsverhältnis Adsorbens/Extrakt von 1/30 bis 1/50 in Berührung bringt.

4. Verfahren nach Anspruch 2, dadurch gekennzeichnet, daß man den von Purinen befreiten wäßrigen Extrakt bei einem Unterdruck von 700 bis 740 mm Quecksilber und bei einer Temperatur von 40 bis 60 °C konzentriert, bis man ein Volumen erhält, das 1/40 bis 1/20 des Ausgangsvolumens beträgt.

5. Verfahren nach Anspruch 4, dadurch gekennzeichnet, daß man die getrockneten Bohnen in Stufe c) durch Zusammenbringen mit einem von Purinen befreiten konzentrierten Extrakt für 8 bis 12 h bei einer Temperatur von 20 bis 30 °C rehydratisiert.

6. Verfahren nach Anspruch 5, dadurch gekennzeichnet, daß man die rehydratisierten Bohnen für 1 bis 2 h bei einer Temperatur von 70 bis 80 °C teilweise trocknet.

7. Verwendung des bei der Durchführung des Verfahrens nach einem der Ansprüche 1 bis 6 erhaltenen Produkts zur Herstellung von Schokolade.